# EUROPÄISCHE PATENTSCHRIFT

(11) **EP 1 389 979 B1**
(45) Veröffentlichungstag und Bekanntmachung des Hinweises auf die Patenterteilung: **06.12.2006**
(21) Anmeldenummer: 02754564.9
(22) Anmeldetag: 07.05.2002
(51) Int. Cl.: A61F 2/28

(54) **KNOCHENIMPLANTAT**
BONE IMPLANT
IMPLANT OSSEUX

(30) Priorität: 29.05.2001 DE 10126085
(43) Veröffentlichungstag der Anmeldung: 25.02.2004
(73) Patentinhaber: Tutogen Medical GmbH, 91077 Neunkirchen am Brand (DE)
(72) Erfinder: LANDIS, Klaus, 90562 Heroldsberg (DE)
(74) Vertreter: Manitz, Finsterwald & Partner GbR
(86) Internationale Anmeldenummer: PCT/EP2002/005032
(87) Internationale Veröffentlichungsnummer: WO 2002/096324

(56) Entgegenhaltungen:
- EP-A- 0 979 638
- WO-A-00/74608
- WO-A-01/08611
- WO-A-01/49219
- DE-A- 2 906 650
- DE-A- 3 933 459
- DE-A- 19 952 550
- DE-C- 19 610 715
- FR-A- 2 552 659
- US-A- 4 190 079
- US-A- 5 112 354
- US-A- 5 171 327
- US-A- 5 246 530
- US-A- 5 348 788
- US-A- 5 769 897

## Beschreibung

Die vorliegende Erfindung betrifft ein Knochenimplantat, insbesondere ein Knochenimplantat aus spongiösem und/oder kortikalem Knochenmaterial menschlichen oder tierischen Ursprungs.

Knochenimplantate der oben genannten Art sind grundsätzlich bekannt. Diese aus menschlichen oder tierischen Knochen gewonnenen Implantate zeichnen sich dadurch aus, daß sie bei Implantation in den menschlichen Körper zum einen ein gutes Anwachsen des Gewebes ermöglichen, in das sie implantiert werden, und zum anderen langsam und vollständig von dem Körper, in den sie implantiert wurden, bei gleichzeitigem Aufbau körpereigenen Knochengewebes abgebaut werden. Der Bereich, in dem Implantat und Körpergewebe miteinander in Kontakt sind, d.h. die Anwachs- und Umbauzone, bildet jedoch grundsätzlich im Vergleich mit dem Knochenmaterial des Implantats wie auch mit dem des menschlichen Körpers mechanisch gesehen eine Schwachstelle.

In US 5 112 354 ist ein texturierter, entmineralisierter und einheitlicher Abschnitt eines Säugetierknochens beschrieben, der als steifes Kollagengerüst mit kleinen Öffnungen für allogenen Skelettwiederaufbau dient. Das Allograft wird durch Behandlung eines Abschnitts eines Knochens zur Entfernung von weichem Gewebe, Texturierung der Knochenoberfläche zur Bereitstellung eines Musters von Löchern vorgegebener Größe, Dichte und Tiefe und abschließende Entmineralisierung des Knochenabschnitts hergestellt, so dass ein steifes, unlösliches Kollagengerüst verbleibt, das für Osteoinduktion nach Implantation geeignet ist. Alle diese Schritte werden unter minimaler Denaturierung der extrazellulären Matrixproteine durchgeführt, die an das Kollagengerüst gebunden bleiben und die zum Abschluss des Prozesses der Knochenneubildung notwendig sind.

Es ist Aufgabe der vorliegenden Erfindung, ein Knochenimplantat der oben genannten Art bereitzustellen, das nach Implantation in den menschlichen Körper zu einer Anwachs- und Umbauzone mit verbesserten mechanischen Eigenschaften führt.

Die Aufgabe wird gelöst durch ein Knochenimplantat mit den Merkmalen nach Anspruch 1.

Ein erfindungsgemäßes Knochenimplantat ist aus spongiösem und/oder kortikalem Knochenmaterial menschlichen oder tierischen Ursprungs gebildet, wobei bei Verwendung verschiedener Knochenmaterialien diese schichtartig oder in der Form von Verbundmaterialien, z.B. mit einer spongiösen Matrix und eingelagerten verstärkenden kortikalen Einlagerungen gebildet sein können.

Zumindest ein Bereich der Oberfläche eines erfindungsgemäßen Knochenimplantats weist mehrere längliche, nicht das Knochenimplantat durchdringende, benachbart angeordnete Bohrungen auf. Hierbei werden unter Bohrungen mechanisch, z.B. durch Bohren oder Fräsen, hergestellte Grundlöcher verstanden, deren Querschnitt nicht unbedingt kreisförmig sein muß. Dieser mit Bohrungen versehene Öberflächenbereich bildet eine vergrößerte Kontaktfläche, an der nach Implantation körpereigenes Gewebe anliegt, was zu einer verbesserten Einheilung von daran anliegendem Gewebe und einem beschleunigten Umbau des Knochenimplantats im Vergleich zur Verwendung eines an seiner Oberfläche kompakten Knochenimplantats ohne Bohrungen führt. Darüber hinaus bildet sich nach der Implantation bei dem Einwachsen von körpereigenem Gewebe in die Bohrungen ein miteinander verzahntes Gebilde aus dem Knochenimplantat mit seinen Bohrungen und den diese begrenzenden Wänden und dem fingerartig in diese Bohrungen einwachsenden körpereigenem Knochengewebe. Während das Material zwischen den Bohrungen auch in der Anfangsphase des Abbaus noch eine große mechanische Festigkeit senkrecht und parallel zu der Richtung der Bohrungen aufweist, können von den in die Bohrungen eingewachsenen Gewebeteilen ebenfalls schon erhebliche Kräfte aufgenommen werden, was insgesamt zu verbesserten mechanischen Eigenschaften in der Anwachs- und Umbauzone führt.

Weiterhin können die Bohrungen während der Implantation dazu verwendet werden, mit entsprechend ausgebildeten Halte- und/oder Positionierwerkzeugen das Implantat zu greifen, zu bewegen und dann zu positionieren. Dadurch ist möglich, das Implantat mit nur minimalem Spiel in eine Öffnung eines Knochens einzusetzen.

Der Oberflächenbereich mit Bohrungen erstreckt sich bis an einen Rand des Knochenimplantats, wobei sich die Bohrungen in dem Randbereich nach außen öffnen und dadurch einen riffelartigen Abschnitt bilden. Diese in bezug auf den mit Bohrungen versehenen Oberflächenbereich seitliche Riffelung ermöglicht auch ein gutes seitliches Einwachsen des Knochenimplantats und erlaubt es darüber hinaus, das Implantat z.B. mit einer Pinzette im Bereich der Riffelung sehr sicher zu greifen, so daß es auch bei nur geringem Kraftaufwand sicher manipulierbar ist. Diese riffelartige Oberfläche kann dadurch erzielt werden, daß nach Einbringen der Bohrungen das Knochenimplantat in dem die Bohrungen tragenden Oberflächenbereich in einer Richtung schräg oder senkrecht zu diesem Oberflächenbereich geschnitten wird.

Vorteilhafte Ausführungsformen der Erfindung sind in der Beschreibung, den Figuren und den Unteransprüchen beschrieben.

Form und Größe eines erfindungsgemäßen Knochenimplantats richten sich nach dem Einsatzzweck und hängen von den Dimensionen des Bereichs ab, in den es eingesetzt werden soll. Insbesondere kann es stift-, platten- oder auch scheibenförmig ausgebildet sein. Selbstverständlich sind auch gewölbte Strukturen möglich. Bevorzugt sind Höhe, Breite und/oder Länge des Implantats im Bereich von etwa 2 mm oder größer.

Bevorzugt sind die Bohrungen matrixartig, d.h. in einem regelmäßigen, bevorzugt rechtwinkligen, Muster angeordnet. Eine solche Anordnung der Bohrungen ermöglicht nicht nur eine einfachere Herstellung des erfindungsgemäßen Knochenimplantats, sondern ermöglicht auch durch die gleichmäßige Verteilung der Bohrungen eine bessere Verteilung von Kräften, die im implantierten Zustand auf den mit Bohrungen versehenen Oberflächenbereich einwirken, sowie eine im allgemeinen höhere Dichte von Bohrungen.

Bevorzugt bildet der Bereich mit den Bohrungen einen Membranabschnitt auf dem Knochenimplantat, dessen Dicke durch die Tiefe der Bohrlöcher bestimmt wird, die hierbei vorzugsweise im wesentlichen gleich lang sind. Auch hierdurch wird eine günstige Verteilung von Kräften auf das Implantat im implantierten Zustand sowie ein möglichst gleichmäßiges Einwachsen des Körpergewebes in die Bohrungen ermöglicht.

Bevorzugt ist der Abstand benachbarter Bohrungen nicht größer als das Zweifache von deren maximaler Weite. Unter dem Abstand zweier Bohrungen wird hierbei der minimale Abstand zwischen zwei Wandbereichen benachbarter Bohrungen verstanden. Hierdurch wird eine vergleichsweise hohe Dichte der Bohrungen und damit der für das Einwachsen und den damit einhergehenden bzw. folgenden Abbau zur Verfügung stehenden Fläche erreicht. Obwohl es grundsätzlich möglich ist, daß Bohrungen direkt aneinandergrenzen, so daß eine Anordnung nicht aneinanderstoßender, durch die Bohrung getrennter Säulen entsteht, ist besonders bevorzugt der Abstand der Bohrungen größer als 0,01 mm, um eine hinreichende mechanische Festigkeit zu erreichen. Besonders bevorzugt liegt der Abstand der Bohrungen zwischen 0,01 mm und der maximalen Weite der Bohrungen.

Der Oberflächenbereich kann dabei bevorzugt zwischen etwa 20 und 200 Bohrungen pro cm² aufweisen.

Die maximale Weite der Bohrungen liegt bevorzugt zwischen etwa 0,05 mm und etwa 2 mm, was zum einen eine einfache Herstellung erlaubt, zum anderen aber eine hohe Dichte von Bohrungen bezogen auf die Oberfläche des Knochenimplantats ermöglicht. Darüber hinaus werden enge Bohrungen schneller von einwachsendem Knochengewebe ausgefüllt, so daß sich schneller eine mit dem Implantat verzahnte, tragfähige Struktur aus körpereigenem Knochengewebe bildet, wodurch eher eine hohe Festigkeit der Anwachs- und Umbauzone erreicht wird. Dabei ist es insbesondere auch möglich, daß der Oberflächenbereich Bohrungen unterschiedlicher maximaler Weite aufweist, wobei die verschiedenen maximalen Weiten so gewählt sind, daß eine besonders hohe Dichte von Bohrungen bezogen auf die mit Bohrungen versehene Fläche des Knochenimplantats und/oder eine besonders hohe Festigkeit dieses Bereichs erzielt wird.

Die Bohrungen sind erfindungsgemäß länglich, was bedeutet, daß die Tiefe einer Bohrung größer als deren maximale Weite ist. Hierbei ist eine die maximale Weite der Bohrung deutlich, d.h. insbesondere um das dreifache, übersteigende Tiefe der Bohrung besonders bevorzugt, da dadurch ein besonders gutes Ineinandergreifen von Knochenimplantat und eingewachsenen Gewebe und damit eine besonders hohe mechanische Festigkeit der Anwachs- und Umbauzone erreicht wird. Besonders bevorzugt kann unter der Maßgabe, daß die Bohrungen länglich sein müssen, die Tiefe der Bohrung zwischen etwa 0,1 mm und etwa 10 mm liegen. Die Tiefe der Bohrungen muß dabei jedoch nicht unbedingt für alle Bohrungen eines Oberflächenbereichs gleich groß sein.

Obwohl die Längsachsen der Bohrungen nicht notwendig parallel ausgerichtet sein müssen, ist es bevorzugt, daß die Achsen der Bohrungen im wesentlichen parallel ausgerichtet sind. Dies ermöglicht nicht nur eine einfachere Herstellung, sondern auch eine wesentlich höhere Dichte an Bohrungen sowie eine gleichmäßigere Lastverteilung. Darüber hinaus können in solche Bohrungen auch leichter Halte- und Positionierungswerkzeuge eingeführt werden. Dabei sind die Bohrungen bevorzugt senkrecht zur Fläche des Knochenimplantats in dem Bohrungen tragenden Oberflächenbereich ausgerichtet, so daß die Wände zwischen den Bohrungen senkrecht auf die Fläche wirkende Kräfte gut aufnehmen können. Ist je nach Implantat eine andere Belastungsrichtung zu erwarten, können die Bohrungen natürlich auch schräg gegenüber der Oberfläche ausgerichtet sein.

Obwohl der Oberflächenbereich des Knochenimplantats, der die Bohrungen beinhaltet, grundsätzlich auch aus spongiösem Material bestehen kann, ist er bevorzugt durch kompaktes Knochenmaterial gebildet, d.h., daß das Knochenmaterial zwischen den Bohrungen kompakt ist. Hierdurch wird eine besonders hohe Festigkeit der die mechanischen Belastungen tragenden Wände zwischen den Bohrungen erreicht.

Bevorzugt erstreckt sich der Oberflächenbereich mit Bohrungen bis an einen Rand des Knochenimplantats, wobei sich die Bohrungen in dem Randbereich nach außen öffnen und dadurch einen riffelartigen Abschnitt bilden. Diese in bezug auf den mit Bohrungen versehenen Oberflächenbereich seitliche Riffelung ermöglicht auch ein gutes seitliches Einwachsen des Knochenimplantats und erlaubt es darüber hinaus, das Implantat z.B. mit einer Pinzette im Bereich der Riffelung sehr sicher zu greifen, so daß es auch bei nur geringem Kraftaufwand sicher manipulierbar ist. Diese riffelartige Oberfläche kann dadurch erzielt werden, daß nach Einbringen der Bohrungen das Knochenimplantat in dem die Bohrungen tragenden Oberflächenbereich in einer Richtung schräg oder senkrecht zu diesem Oberflächenbereich geschnitten wird.

Bevorzugt kann das erfindungsgemäße Knochenimplantat mindestens einen weiteren Oberflächenbereich aufweisen, in den weitere Bohrungen eingebracht sind, die jeweils mindestens eine der Bohrungen in dem Oberflächenbereich wenigstens teilweise schneiden. Hierbei kann es sich bei dem weiteren Oberflächenbereich grundsätzlich auch um den Oberflächenbereich mit Bohrungen handeln, besonders bevorzugt ist jedoch ein weiterer Oberflächenbereich, der gegen den Oberflächenbereich geneigt ist. Weiterhin müssen die Bohrungen sich nicht unbedingt über ihren ganzen Querschnitt durchdringen, vielmehr genügt es grundsätzlich, daß die Bohrungen im Schnittbereich verbunden sind. Bei dieser Weiterbildung bilden die sich gegebenenfalls nur teilweise schneidenden Bohrungen gewinkelte Kanäle, die beim Einwachsen von Gewebe eine besonders gute Verbindung zwischen Implantat und einwachsendem Gewebe mit sich bringen. Insbesondere ergibt sich auch eine gute Aufnahme von Zugkräften, die auf das Implantat in der Richtung der Bohrungen wirken.

Durch die weiterein Bohrungen wird das Implantat im Rahmen der typischerweise auftretenden Kräfte nicht signifikant geschwächt.

Besonders bevorzugt schneidet wenigstens eine der weiteren Bohrungen in dem weiteren Oberflächenbereich wenigstens eine der Bohrungen in dem Oberflächenbereich in einem Winkel von etwa 90°, da hierdurch besonders gut Kräfte in Richtung der Bohrungen des Oberflächenbereichs durch in die weiteren Bohrungen eingewachsenes Gewebe aufgenommen werden können.

Weiterhin sind besonders bevorzugt wenigstens ein Teil der weiteren Bohrungen und ein Teil der diese schneidenden Bohrungen in dem Oberflächenbereich so ausgebildet, daß diese ein Gitter von miteinander verbundenen Bohrkanälen bilden. Besonders vorteilhaft sind mehrere so entstehenden Gitter in einer dritten Raumdimension benachbart angeordnet. Das sich so bildende, vorzugsweise regelmäßige, Gitter von miteinander verbundenen Bohrungen bildet zum einen eine besonders große Oberfläche zum Einwachsen von körpereigenem Gewebe. Zum anderen kann der Einwachsbereich, das heißt der mit Bohrungen und/oder weiteren Bohrungen versehene Bereich des Implantats und in die Bohrungen bzw. weiteren Bohrungen eingewachsenes körpereigenes Gewebe durch die Interprenetation von zusammenhängendem Implantatmaterial zwischen den Bohrungen und/oder den weiteren Bohrungen und in das Gitter von verbundenen Bohrungen eingewachsendem, zusammenhängendem Gewebe besonders gut Kräfte zwischen Implantat und körpereigenem Gewebe übertragen, so daß der Einwachsbereich mechanisch besonders stabil ist.

Weiterhin kann auch ein System von Bohrungen vorgesehen sein, bei dem sich mindestens drei nicht kollineare Bohrungen wenigstens teilweise schneiden, wodurch sich ein dreidimensionales System von verbundenen Kanälen ergibt.

Das Material des Knochenimplantats besteht bevorzugt aus konserviertem und sterilem Knochenmaterial. Hierdurch ist das Knochenimplantat lagerfähig und kann ohne das Risiko von Infektionen implantiert werden.

Wegen der besseren Verfügbarkeit ist das Knochenimplantat bevorzugt tierischen Ursprungs. Aufgrund der hohen Festigkeit von Rinderknochen ist dabei bovines Knochenmaterial besonders bevorzugt.

Die Herstellung eines erfindungsgemäßen Implantats kann von einem Stück eines geeigneten Knochenmaterials ausreichender Größe ausgehen, das dann in mehreren Schritten weiter prozessiert wird. Die Herstellung der Bohrungen kann grundsätzlich vor, zwischen oder nach den Schritten erfolgen. Nach einem Verfahren kann natives Knochenmaterial zunächst zum Beispiel in Salzlösung gereinigt und dann bekannten Verfahren entfettet werden. Nach einem Inaktivierungs- und Denaturierungsschritt wird das Material konserviert.

Die Konservierung des Knochenmaterials kann beispielsweise mittels Gefriertrocknung erfolgen. Bevorzugt wird jedoch eine Konservierung durch Lösemitteldehydratisierung von nativem Knochenmaterial mittels eines organischen, mit Wasser mischbaren Lösungsmittels, z.B. Methanol, Ethanol, Propanol, Isopropanol, Aceton, Methylethylketon oder Mischungen dieser Lösungsmittel durchgeführt.

Danach wird der so entstandene Rohling zugerichtet, verpackt und gegebenenfalls sterilisiert.

Die Sterilisation kann insbesondere durch Bestrahlen mit Gamma- und/oder Elektronenstrahlen nach der Lösemitteldehydratisierung erfolgen. Die Konservierung und Sterilisation unter Verwendung der Lösemitteldehydratisierung ist in dem Patent DE 29 06 650 beschrieben. Spongiöses Knochenmaterial kann alternativ auch aseptisch prozessiert werden, so daß eine separate Sterilisation nicht mehr notwendig ist.

Zur Bearbeitung können reinraumspezifische CNC-Fräs-Bohrmaschinen und dafür geeignete Werkzeuge verwendet werden.

Nachfolgend wird die vorliegende Erfindung beispielhaft anhand von zwei vorteilhaften Ausführungsformen unter Bezugnahme auf die beigefügten Zeichnungen beschrieben. Es zeigen
- Fig. 1: eine schematische, perspektivische Ansicht eines stiftförmigen Knochenimplantats nach einer ersten bevorzugten Ausführungsform der Erfindung,
- Fig. 2: eine schematische, perspektivische Ansicht eines stiftförmigen Knochenimplantats nach einer zweiten bevorzugten Ausführungsform der Erfindung und
- Fig. 3: eine Schnittansicht durch das Knochenimplantat in Fig. 2 entlang der in Fig. 2 durch gestrichelte Linien angedeuteten Ebene.

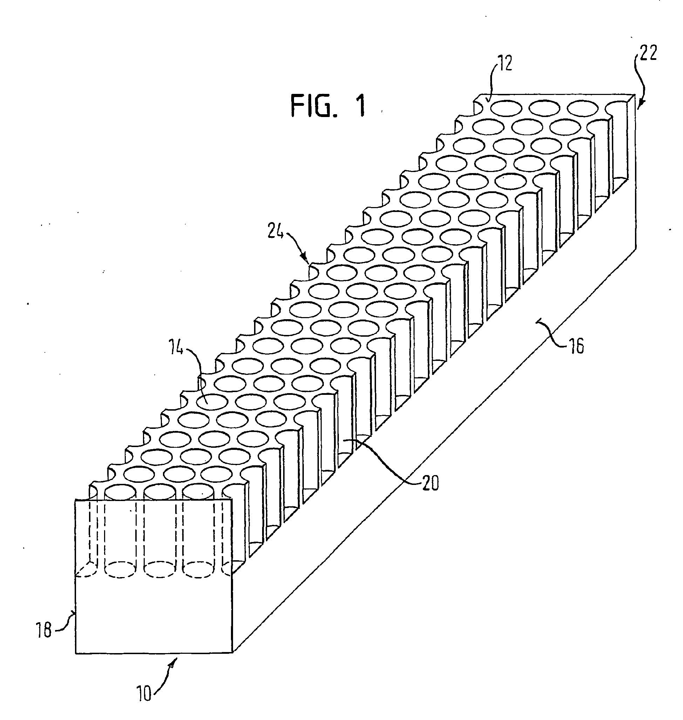

In Fig. 1 weist ein stiftförmiges Knochenimplantat 10 einen Oberflächenbereich 12 mit Bohrungen 14 auf.

Das Knochenimplantat 10 hat die Form eines Quaders, wobei die Höhe und die Breite etwa 4 mm und die Länge etwa 60 mm beträgt. Das Knochenimplantat ist aus konserviertem kortikalem Knochenmaterial von Rindern hergestellt.

Die Bohrungen 14 in dem Oberflächenbereich 12 des Knochenimplantats 10 sind zylindrisch und weisen einen Durchmesser bzw. eine maximale Weite von etwa 0,8 mm auf. Die Bohrungen sind auf einem quadratischen Gitter angeordnet, wobei sie einen Abstand von etwa 0,2 mm haben. Dies resultiert in einer Oberflächendichte von etwa 100 Bohrungen pro cm² der Oberfläche 12.

Die zylindrisch ausgebildeten Bohrungen 14 sind parallel und senkrecht zu dem Oberflächenbereich 12 ausgerichtet und weisen eine Tiefe von etwa 2 mm auf, so daß die die Bohrungen enthaltende Schicht einen membranartigen Abschnitt von etwa 2 mm Dicke entlang des Oberflächenbereichs 12 mit Bohrungen 14 aufweist.

Wie in der Figur gezeigt erstreckt sich der Oberflächenbereich 12 mit Bohrungen 14 bis an die Ränder des Knochenimplantats. Die Bohrungen (20) in den Randbereichen entlang den Seitenflächen 16 bzw. 18 öffnen sich nach außen, wodurch jeweils ein geriffelter Bereich 22 bzw. 24 mit Ausnehmungen in Form eines Halbzylinders entsteht. Bei einer anderen Ausführungsform kann die Riffelung auch, anders als in der Figur, um den Oberflächenbereich 12 umlaufend ausgebildet sein.

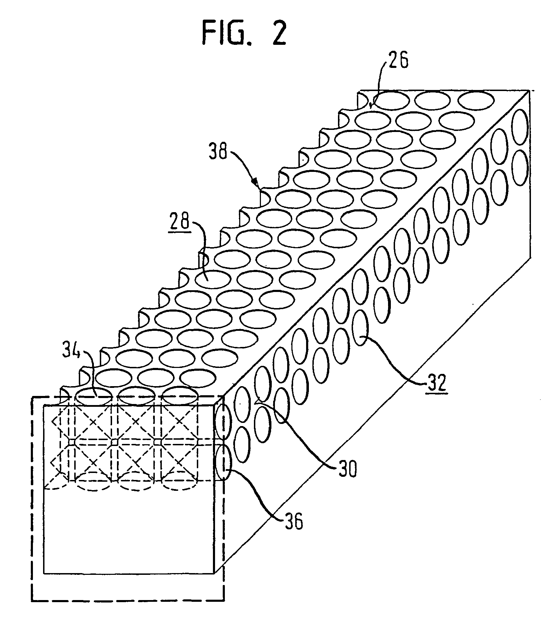

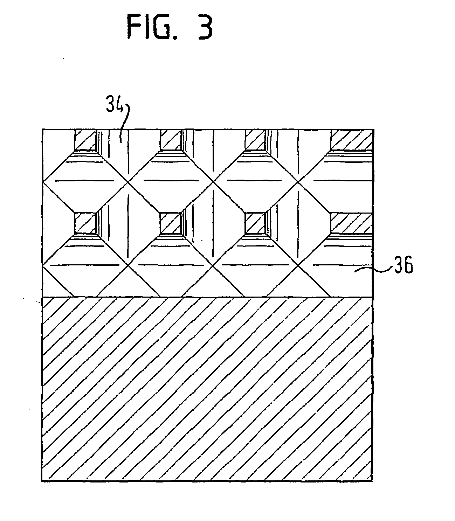

In Fig. 2 weist ein Knochenimplantat nach einer zweiten bevorzugten Ausführungsform in einem Oberflächenbereich 26 senkrecht zu dem Oberflächenbereich verlaufende, zylindrische, in regelmäßigen Abständen matrixartig angeordnete Bohrungen 28 auf. In einem seitlichen, weiteren Oberflächenbereich 30 sind weitere Bohrungen 32 ebenfalls matrixartig angeordnet. Jeweils in einer Zeile angeordnete Bohrungen 34 der Bohrungen 28 schneiden in einer entsprechenden Spalte angeordnete weitere Bohrungen 36 der weiteren Bohrungen 32, wie in Fig. 3 gezeigt, in einem Winkel von etwa 90°. Hierdurch wird in jeder durch eine Zeile und eine zugehörige Spalte definierten Ebene ein gitterartiges System von miteinander verbundenen Kanälen gebildet, so daß eine der Anzahl der Zeilen und Spalten entsprechende Anzahl im wesentlichen paralleler Systeme von gitterartig verbundenen Kanälen in dem Implantat ausgebildet ist.

Eine Längsseite des Implantats nach der zweiten Ausführungsform weist einen geriffelten Bereich 38 auf, der entsprechend den geriffelten Bereichen bei dem ersten Ausführungsbeispiel ausgebildet ist, aber zusätzlich die in den Figuren nicht sichtbaren Öffnungen der weiteren Bohrungen 32 enthält.

### Bezugszeichenliste

- 10: Knochenimplantat
- 12: Oberflächenbereich
- 14: Bohrungen
- 16: Seitenfläche
- 18: Seitenfläche
- 20: Bohrung
- 22: geriffelter Bereich
- 24: geriffelter Bereich
- 26: Oberflächenbereich
- 28: Bohrungen
- 30: weiterer Oberflächenbereich
- 32: weitere Bohrungen
- 34: Bohrungen
- 36: weitere Bohrungen
- 38: geriffelter Bereich

## Patentansprüche

1. Knochenimplantat aus spongiösem und/oder kortikalem Knochenmaterial menschlichen oder tierischen Ursprungs,
bei dem zumindest ein Oberflächenbereich (12; 26, 30) des Knochenimplantats (10) mehrere längliche, nicht das Knochenimplantat durchdringende, benachbart angeordnete Bohrungen (14; 28, 32) aufweist,
**dadurch gekennzeichnet,**
**daß** sich der Oberflächenbereich (12; 26) mit Bohrungen (14; 28) bis an einen Rand des Knochenimplantats erstreckt, wobei sich die Bohrungen (20) in dem Randbereich nach außen öffnen und dadurch einen riffelartigen Abschnitt (22, 24) bilden.

2. Knochenimplantat nach Anspruch 1,
**dadurch gekennzeichnet,**
**daß** die Bohrungen (14; 28, 34) matrixartig angeordnet sind.

3. Knochenimplantat nach Anspruch 1 oder 2,
**dadurch gekennzeichnet,**
**daß** der Oberflächenbereich (12; 26) mit den Bohrungen (14; 28) einen Membranabschnitt bildet.

4. Knochenimplantat nach einem der vorhergehenden Ansprüche,
**dadurch gekennzeichnet,**
**daß** die maximale Weite der Bohrungen (14; 28) zwischen etwa 0,05 mm und etwa 2 mm liegt.

5. Knochenimplantat nach einem der vorhergehenden Ansprüche,
**dadurch gekennzeichnet,**
**daß** die Tiefe der Bohrungen (14; 28) zwischen etwa 0,1 mm und etwa 10 mm liegt.

6. Knochenimplantat nach einem der vorhergehenden Ansprüche,
**dadurch gekennzeichnet,**
**daß** der Abstand benachbarter Bohrungen (14; 28) nicht größer als das zweifache von deren maximaler Weite ist.

7. Knochenimplantat nach einem der vorhergehenden Ansprüche,
**dadurch gekennzeichnet,**
**daß** der Oberflächenbereich (12; 26) zwischen etwa 20 und 200 Bohrungen (14; 28) pro cm² aufweist.

8. Knochenimplantat nach Anspruch 4,
**dadurch gekennzeichnet,**
**daß** der Abstand der Bohrungen (14; 28) zwischen 0,01 mm und der maximalen Weite der Bohrungen liegt.

9. Knochenimplantat nach Anspruch 5,
**dadurch gekennzeichnet,**
**daß** die Achsen der Bohrungen (14; 28) im wesentlichen parallel ausgerichtet sind.

10. Knochenimplantat nach einem der vorhergehenden Ansprüche,
**dadurch gekennzeichnet,**
**daß** das Knochenmaterial zwischen den Bohrungen (14) kompakt ist.

11. Knochenimplantat nach einem der vorhergehenden Ansprüche,
**dadurch gekennzeichnet,**
**daß** das Knochenimplantat einen weiteren Oberflächenbereich (30) aufweist, in den weitere Bohrungen (32) eingebracht sind, die jeweils mindestens eine der Bohrungen (28) in dem Oberflächenbereich (26) wenigstens teilweise schneiden.

12. Knochenimplantat nach Anspruch 11,
**dadurch gekennzeichnet,**
**daß** wenigstens eine der weiteren Bohrungen (32) in dem weiteren Oberflächenbereich (30) wenigstens eine der Bohrungen (28) in dem Oberflächenbereich (26) in einem Winkel von etwa 90° wenigstens teilweise schneidet.

13. Knochenimplantat nach Anspruch 11 oder 12,
**dadurch gekennzeichnet,**
**daß** wenigstens ein Teil der weiteren Bohrungen (32) und ein Teil der diese kreuzenden Bohrungen (28) in dem Oberflächenbereich (26) so ausgebildet sind, daß diese ein Gitter von miteinander verbundenen Bohrkanälen (34, 36) bilden.

14. Knochenimplantat nach einem der vorhergehenden Ansprüche,
**dadurch gekennzeichnet,**
**daß** das Material des Knochenimplantats aus konserviertem und sterilem Knochenmaterial besteht.

## Claims

1. A bone implant of spongious and/or cortical bone material of human or animal origin,
in which at least one surface region (12: 26, 30) of the bone implant (10) has a plurality of elongate bores (14; 28, 32) arranged adjacently and not passing through the bone implant
**characterized in that**
the surface region (12; 26) with bores (14; 28) extends up to a margin of the bone implant, with the bores (20) in the marginal region being outwardly open and thereby forming a ribbed section (22, 24).

2. A bone implant in accordance with claim 1, **characterized in that** the bores (14; 28, 34) are arranged in a matrix form.

3. A bone implant in accordance with claim 1 or claim 2, **characterized in that** the surface region (12; 26) with the bores (14; 28) forms a membrane section.

4. A bone implant in accordance with any one of the preceding claims, **characterized in that** the maximum width of the bores (14; 28) lies between approximately 0.05 mm and approximately 2 mm.

5. A bone implant in accordance with any one of the preceding claims, **characterized in that** the depth of the bores (14; 28) lies between approximately 0.1 mm and approximately 10 mm.

6. A bone implant in accordance with any one of the preceding claims, **characterized in that** the spacing of adjacent bores (14; 28) is not larger than twice their maximum width.

7. A bone implant in accordance with any one of the preceding claims, **characterized in that** the surface region (12; 26) has between approximately 20 and 200 bores (14; 28) per cm².

8. A bone implant in accordance with claim 4, **characterized in that** the spacing of the bores (14; 28) lies between 0.01 mm and the maximum width of the bores.

9. A bone implant in accordance with claim 5, **characterized in that** the axes of the bores (14; 28) are aligned substantially parallel.

10. A bone implant in accordance with any one of the preceding claims, **characterized in that** the bone material between the bores (14) is compact.

11. A bone implant in accordance with any one of the preceding claims, **characterized in that** the bone implant has a further surface region (30) into which further bores (32) have been introduced which each at least partly intersect at least one of the bores (28) in the surface region (26).

12. A bone implant in accordance with claim 11, **characterized in that** at least one of the further bores (32) in the further surface region (30) at least partly intersects at least one of the bores (28) in the surface region (26) at an angle of approximately 90°.

13. A bone implant in accordance with claim 11 or claim 12, **characterized in that** at least some of the further bores (32) and some of the bores (28) intersecting them in the surface region (26) are formed such that they form a grid of mutually connected bore passages (34, 36).

14. A bone implant in accordance with any one of the preceding claims, **characterized in that** the material of the bone implant consists of preserved and sterile bone material.

## Revendications

1. Implant osseux en matériau osseux spongieux et/ou cortical d'origine humaine ou animale, dans lequel au moins une région de surface (12 ; 26, 30) de l'implant osseux présente plusieurs perçages (14 ; 28, 32) allongés ne traversant pas l'implant osseux et agencés au voisinage les uns des autres; **caractérisé en ce que** la région de surface (12 ; 26) avec perçages (14 ; 28) s'étend jusqu'à un bord de l'implant osseux, les perçages (20) s'ouvrant vers l'extérieur dans la région de bord et formant ainsi un tronçon cannelé (22, 24).

2. Implant osseux selon la revendication 1, **caractérisé en ce que** les perçages (14 ; 28, 34) sont agencés à la manière d'une matrice.

3. Implant osseux selon la revendication 1 ou 2, **caractérisé en ce que** la région de surface (12 ; 26) avec les perçages (14 ; 28) forme un tronçon de membrane.

4. Implant osseux selon l'une des revendications précédentes, **caractérisé en ce que** la largeur maximale des perçages (14 ; 28) est entre environ 0,05 mm et environ 2 mm.

5. Implant osseux selon l'une des revendications précédentes, **caractérisé en ce que** la profondeur des perçages (14 ; 28) est entre environ 0,1 mm et environ 10 mm.

6. Implant osseux selon l'une des revendications précédentes, **caractérisé en ce que** la distance entre des perçages (14 ; 28) voisins n'est pas supérieure au double de leur largeur maximale.

7. Implant osseux selon l'une des revendications précédentes, **caractérisé en ce que** la région de surface (12 ; 26) présente entre environ 20 et 200 perçages (14 ; 28) par cm².

8. Implant osseux selon la revendication 4, **caractérisé en ce que** la distance entre les perçages (14 ; 28) est entre 0,01 mm et la largeur maximale des perçages.

9. Implant osseux selon la revendication 5, **caractérisé en ce que** les axes des perçages (14 ; 28) sont orientés sensiblement en parallèle.

10. Implant osseux selon l'une des revendications précédentes, **caractérisé en ce que** le matériau osseux entre les perçages (14) est compact.

11. Implant osseux selon l'une des revendications précédentes, **caractérisé en ce que** l'implant osseux présente une autre région de surface (30) dans laquelle sont ménagés d'autres perçages (32) qui coupent chacun au moins partiellement au moins l'un des perçages (28) dans la région de surface (26).

12. Implant osseux selon la revendication 11, **caractérisé en ce qu'**au moins un des autres perçages (32) dans l'autre région de surface (30) coupe au moins partiellement au moins un des perçages (28) dans la région de surface (26) sous un angle d'environ 90°.

13. Implant osseux selon la revendication 11 ou 12, **caractérisé en ce qu'**au moins une partie des autres perçages (32) et une partie des perçages (28) croisant ceux-ci dans la région de surface (26) sont réalisés de telle sorte que ceux-ci forment une grille de canaux percés (34, 36) reliés les uns aux autres.

14. Implant osseux selon l'une des revendications précédentes, **caractérisé en ce que** le matériau de l'implant osseux est constitué par du matériau osseux conservé et stérile.
